(19) Europäisches Patentamt
European Patent Office
Office européen des brevets

(11) Veröffentlichungsnummer: **0 011 761**
**A2**

(12) # EUROPÄISCHE PATENTANMELDUNG

(21) Anmeldenummer: **79104446.4**

(22) Anmeldetag: **12.11.79**

(51) Int. Cl.³: **A 61 K 6/00**

(30) Priorität: **24.11.78 DE 2850916**

(43) Veröffentlichungstag der Anmeldung:
**11.06.80 Patentblatt 80/12**

(84) Benannte Vertragsstaaten:
**AT BE CH DE FR GB IT LU NL SE**

(71) Anmelder: **BAYER AG**
**Zentralbereich Patente, Marken und Lizenzen**
**D-5090 Leverkusen 1, Bayerwerk(DE)**

(72) Erfinder: **Walkowiak, Michael, Dr.**
**Albertus Magnus Strasse 10**
**D-5090 Leverkusen 1(DE)**

(72) Erfinder: **Podszun, Wolfgang, Dr.**
**Wolfskaul 4**
**D-5000 Köln 80(DE)**

(72) Erfinder: **Leusner, Bernhard Wilhelm, Dr.**
**Grüner Weg 148**
**D-5090 Leverkusen(DE)**

(72) Erfinder: **Süling, Carlhans, Dr.**
**Carl-Leverkus-Strasse 10**
**D-5068 Odenthal(DE)**

(72) Erfinder: **Schulz, Hans-Hermann, Dr.**
**Am Neulandkreuz 23**
**D-5653 Leichlingen(DE)**

(54) **Dentalwerkstoffe auf Basis von organischen Kunststoffen in pastöser Form.**

(57) Dentalwerkstoffe auf Basis von organischen Kunststoffen in pastöser Form, in denen mindestens einer Phase aus feinverteilten, vernetzten Kunststoffen besteht, die zu mindestens 50 % copolymerisierte Ester der Methacrylsäure enthalten, und die zweite Phase zu mindestens 50 % aus Estern der Methacrylsäure besteht.

Diese Pasten, vor allem diejenigen, bei denen die feinverteilten, vernetzten Kunststoffe Polymerisatperlen mit einer mittleren Teilchengrößen von 10 - 100 µ sind, eignen sich hervorragend als Zahnfüllmaterial.

EP 0 011 761 A2

BAYER AKTIENGESELLSCHAFT 509 Leverkusen, Bayerwerk

Zentralbereich
Patente, Marken und Lizenzen Si-by

# Dentalwerkstoffe auf Basis von organischen Kunststoffen in pastöser Form

Die Verwendung von Kunststoffen als Werkstoff für Prothesen, Kronen, Brücken, künstlichen Zähnen und Zahnfüllungen ist allgemein bekannt. Organische Materialien für Zahnfüllungen werden üblicherweise in Form von Zubereitungen aus Polymerisat und polymerisierbarem Binder angewendet. Diese Mischungen haben auf Grund ihrer Konsistenz und Klebrigkeit anwendungstechnische und klinische Nachteile.

Die bisher üblichen organischen Materialien werden auf Grund ihrer Konsistenz in die Kavität eingestrichen. Häufig werden die eingebrachten Massen, bedingt durch das Haften am Füllinstrument, teilweise nach dem Einfüllen in die Kavität von der Kavitätenwandung abgezogen. Diese Erscheinung ist in der Regel vom Zahnarzt nicht feststellbar und führt daher zu nicht wandständigen inkompletten Füllungen mit den bekannten Nachteilen.

Besonders nachteilig wirkt sich das verstärkte Kleben der bisher bekannten Füllmaterialien bei mehrflächigen Kavitäten aus. So ist, wie aus der Amalgamfülltechnik her bekannt, ein einwandfreies Ausfüllen der Kavität nur möglich, wenn ein Füllmaterial portionsweise eingebracht wird. Bei dieser Fülltechnik werden zunächst kleine Portionen wandständig in die Kavitätenwinkel gestopft und anschließend erst die Kavität ausgefüllt.

Um den hohen Polymerisationsschwund der bisherigen organischen Füllmaterialien auszugleichen war es erforderlich, das Material portionsweise in der Kavität aushärten zu lassen, eine Technik, die sehr zeitaufwendig ist.

Ein weiterer Nachteil der bisher bekannten organischen Füllmaterialien war der schlechte Verbund mit der Kavitätenwandung. Die Haftung konnte auch nicht wesentlich gesteigert werden durch Anwendung der Säureätzetechnik oder die Anbringung von Retentionsstiften.

Während bei einflächigen Füllungen im Frontzahnbereich die Oberflächengestalt durch Anlegen von Matrizenbändern erzielt wird, bereitet die Formung von okklusalen Flächen mit Materialien, die eine klebende Konsistenz aufweisen, Schwierigkeiten. So war bei der bisher bekannten Materialien eine Formung der Kauflächenbezirke nur in groben Zügen möglich. Nach dem Aushärten war daher üblicherweise eine Formgebung durch rotierende Schleif- und Polierkörper erforderlich. Bekanntlich sind hierbei Verletzungen der benachbarten Schmelzbezirke in der Regel nicht zu vermeiden. Die Folgen hiervon sind Verfälschungen des Kauflächenreliefs und gegebenenfalls okklusale Störungen.

Man hat versucht, die wünschenswerte Oberflächengestalt durch Einstellung einer "schnitzbaren" Eigenschaft zu erzielen. Dieses "schnitzbare" Verhalten tritt jedoch erst dann ein, wenn schon ein gewisser Polymerisationsumsatz erreicht ist. Erfolgt in diesem Zustand die Bearbeitung des Füllmaterials, so kann ein Auf- bzw. Einreißen der Füllungsoberfläche und damit eine Schädigung der Füllung nicht ausgeschlossen werden. Diese Risse, erzielt durch "Schnitzen", können Eintrittspforten für Microorganismen und für Farbstoffe mit den bekannten Auswirkungen sein. Darüber hinaus kann das Bearbeiten von schon anpolymerisierten Materialien zu Polymerisationsstörungen führen.

Gegenstand der Erfindung sind nun Dentalwerkstoffe auf Basis von organischen Kunststoffen in pastöser Form, in denen mindestens einer Phase aus feinverteilten, vernetzten Kunststoffen besteht, die zu mindestens 50 % copolymerisierte Ester der Methacrylsäure enthalten, und die zweite Phase aus polymerisierbaren Bindern besteht, die zu mindestens 50 % Ester der Methacrylsäure enthalten.

Es wurde überraschenderweise gefunden, daß diese Pasten, vor allem diejenigen, bei denen die feinverteilten, vernetzten Kunststoffe Polymerisatperlen mit einer mittleren Teilchengröße von 10 - 100 $\mu$ sind, sich hervorragend als Zahnfüllmaterial eignen.

Die erfindungsgemäßen Materialien lassen sich in einer Konsistenz herstellen, die eine Verarbeitung ermöglicht, wie sie in der Amalgamfülltechnik gebräuchlich ist, das heißt sie lassen sich

a) stopfen
und
b) modellieren.

Zu a)
Mit den erfindungsgemäßen Materialien gelingt es, auf Grund einer nicht klebenden, festen, stopfbaren Konsistenz, ein- und mehrflächige Kavitäten in mehreren Portionen wandständig aufzufüllen. Durch die besondere Eigenschaft des Materials kommt es beim portionsweisen Stopfen nicht zu Schichtenbildungen, das heißt, die einzelnen Portionen verbinden sich homogen miteinander.

Nach dem jeweiligen Einbringen einer Portion in die Kavität und dem Stopfen bzw. Adaptieren bleibt diese, ohne ihre Form zu verändern, am Ort liegen, das heißt, sie läßt sich auch nicht elastisch deformieren.

Die Kavitätenfüllung läßt sich ferner, auf Grund der besonderen Konsistenz, mit sogenannten Amalgampistolen durchführen, ohne daß sich das Füllmaterial wieder von der Kavitätenwandung abzieht oder an der Pistolenöffnung haften bleibt.

Zu b)
Die erfindungsgemäßen Materialien zeigen eine durch Instrumente formbare Konsistenz schon unmittelbar nach dem Anmischvorgang. Diese Konsistenz ermöglicht es, daß nach dem Auffüllen der Kavität, mittels geeigneter Instrumente, z.B. aus Kunststoff oder aus Metall, wie sie in der Amalgamfülltechnik verwendet werden, die okklusale individuelle Kauflächengestalt geformt wird.

Die pastösen erfindungsgemäßen Dentalwerkstoffe auf Basis von organischen Kunststoffen, gehen durch Aushärtung in feste Körper über, die gut polierbar sind.

Zur Herstellung der erfindungsgemäßen Dentalwerkstoffe wurden 40-80, vorzugsweise 50-75 Gew.-Teile feinverteilter vernetzter Kunststoff, 20-60, vorzugsweise 25 bis 50 Gew.-Teile polymerisierbare Binder und 0,01 bis 5 Gew.-Teile Starterzusätze zu einer Paste vermischt. Zur Erleichterung der Pastenfertigung können Inhibitoren, Lichtschutzmittel und/oder bis zu 5 Gew.-Teile amorphe Kieselsäure zugesetzt werden. Für bestimmte Inidkationen kann es angezeigt sein, zusätzlich Farbstoffe anzusetzen.

Als feinverteilter, vernetzter Kunststoff für die erfindungsgemäßen pastösen Zubereitungen eignen sich feste, zerkleinerte, zweckmäßig pulverförmige vernetzte Polymerisate, die mindestens 50 % copolymerisierte Ester der Methacrylsäure, vorzugsweise Methacrylsäuremethylester, enthalten. Das feinteilige, vernetzte Polymerisat kann als Splitterpolymerisat, Emulsionspolymerisat oder besonders günstig als Perlpolymerisat vorliegen, wobei die mittlere Korngröße des Perlpolymerisats vorzugsweise zwischen 10 und 100 µ betragen soll. Als Vernetzer eignen sich die mit Methylmethacrylat copolymerisierbaren Polyvinylverbindungen, wie beispielsweise Äthylenglycoldimethacrylat und Divinylbenzol, wobei der Vernetzeranteil 2 bis 35 Gew.-% des Monomergemisches betragen sollte. Neben dem Vernetzer können weitere Monomere in den organischen Füllstoff einpolymerisiert sein, um beispielsweise das Quellverhalten des Füllstoffs zu beeinflussen oder um die mechanischen Eigenschaften des ausgehärteten Dentalkunststoffs zu modifizieren.

Gut geeignet für die Pastenzubereitung sind auch Perlpolymere mit einem mittleren Durchmesser von 10 bis 200µ aus einem oder mehreren polymerisierten Methacrylaten und/oder Dimethacrylaten mit einer Viskosität von 0,5 bis 500 Pa.s und vorzugsweise bis zu 20 Gew.-% von einem oder mehreren anderen Vinylmonomeren.

Als Ester der Methacrylsäure eignen sich solche von einwertigen und mehrwertigen Alkoholen, gegebenenfalls im Gemisch mit anderen Vinylmonomeren, mit der Auflage, daß der Gehalt an Estern der Methacrylsäure mindestens 50 % sein muß.

Als geeignete Ester der Methacrylsäure seien beispielsweise aliphatische und cycloaliphatische Ester genannt, wie Methylmethacrylat, Äthylmethacrylat und Cyclohexylmethacrylat.

Besonders gut geeignet sind ferner Ester von mehrwertigen Alkoholen, mit einem Molekulargewicht von 190-10000, insbesondere Ester von 2 und 3-wertigen Alkoholen mit einem Molekulargewicht im Bereich von 190 bis 800, wie z.B. Ethylenglykoldimethacrylat, Triethylenglykoldimethacrylat, Neopentlyglykoldimethacrylat oder Trimethylolpropantrimethacrylat, desweiteren Urethan und Ureidpolymethacrylate, die durch Umsetzung von Hydroxyalkylmethacrylat bzw. Aminoalkylmethacrylat mit Polyisocyanaten zugänglich sind, z.B. die Verbindung der Formel

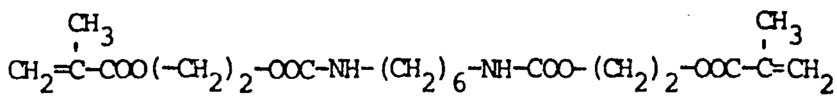

Sehr gute Pasten erhält man, wenn als Ester der Methacrylsäure zumindest in Anteilen Verbindungen vom Typ des Bis-GMA der Formel

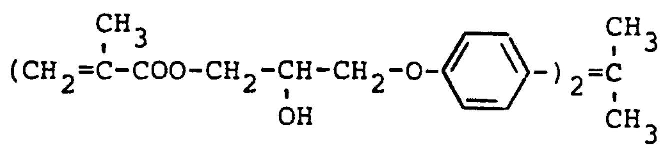

eingesetzt werden.

Zahnfüllmassen mit guter Konsistenz und einem hohen Niveau der mechanischen Festigkeit werden besonders dann erhalten, wenn man als Ester der Methacrylsäure Mischungen aus verschiedenen Methacrylsäureestern ververwendet, z.B. Mischungen von 20-70 Gew.-Teilen Bis GMA und 30-80 Gew.-Teilen Triethylenglykoldimethacrylat.

Als Starterzusätze können die üblichen Startersysteme verwendet werden, d.h. Radikale, Anionen oder Kationen liefernde Systeme, die eine radikalische, anionische oder kationische Polymerisation auslösen können. Im Falle von Radikale liefernden Systemen sind Peroxide oder aliphatische Azoverbindungen besonders geeignet, beispielsweise Benzoylperoxid, Laurylperoxid oder Azoisobuttersäuredinitril, die üblicherweise in Mengen von 0,1 bis 5 Gew.-% eingesetzt werden. Während die Härtung bei erhöhter Temperatur allein durch Peroxide oder andere Radikalstarter durchgeführt wird, ist zur Härtung bei Raumtemperatur ein Zusatz von Beschleunigern, vorzugsweise aromatischen Aminen notwendig. Geeignete Beschleuniger sind N-N-substituierte Toluidine und Xylidine, wie N,N-Dimethyl-p-toluidin oder N,N-Bis-(2-hydroxyethyl)-xylidin. Gute Aushärtung erzielt man mit 0,5-3 % Aminzusatz. Eine günstige Darbietungsform für ein Peroxid/Beschleuniger aktiviertes System ist die 2-Pasten-Form, wobei eine Paste den Radikalstarter und die andere den Beschleuniger enthält und die Aushärtung durch Mischen beider Pasten eingeleitet wird.

Auch eine Aushärtung unter Verwendung von UV-Licht oder sichtbarem Licht bei geeigneter Sensibilisierung ist eine sehr gute Methode. Geeignete Photoinitiatoren sind beispielsweise Benzophenon und seine Derivate, Benzoin und seine Derivate, wie Benzoinether, Anthrachinone und aromatische Disulfide.

## Beispiel 1

Herstellung eines Perlpolymerisats aus Methylmethacrylat und Äthylenglycoldimethacrylat

### Polymerisation

| | |
|---|---|
| Reaktionsgefäß: | 6-Liter-Autoklav mit Doppelankerrührer |
| Lösung I (Dispergatorlösung) : | 2500 ml dest. Wasser<br>500 ml 7,5 %ige wäßrige Lösung des Copolymerisats aus 1 Gew.-Teil Methacrylsäure und 1 Gew.-Teil Methylmethacrylat mit dem pH = 6 und der Viskosität: 3650 cp |
| Lösung II : | 690 g Methylmethacrylat<br>60 g Äthylenglycoldimethacrylat<br>3,75 g Benzoylperoxid<br>3,75 g Lauroylperoxid |

Lösung I wird vorgelegt und 5 Minuten verrührt. Bei stehendem Rührer gibt man Lösung II auf einmal zu und bespült den Autoklaven mit Stickstoff. Anschließend wird 5 bar Stickstoff aufgedrückt, die Rührgeschwindigkeit auf 400 UpM eingestellt und auf 80°C aufgeheizt. Bei einsetzender exothermer Reaktion wird so stark gekühlt, daß die Temperatur unter 90°C bleibt. Man läßt 2 Stunden bei 80°C nachrühren.

Aufarbeitung

Der Ansatz wird abgeblasen und mit destilliertem Wasser auf 10 l verdünnt. Nach der Zugabe von 180 g Eisessig wird 15 Minuten auf 90 - 100 $^{\circ}C$ aufgeheizt. Das ausfallende Perlpolymerisat wird nach Erkalten abgesaugt, durch dreimaliges Aufrühren in je 5 l destilliertem Wasser gewaschen und bei 60 $^{\circ}C$ getrocknet.
Ausbeute: 645 g,
mittlerer Perldurchmesser: 25 $\mu$.

Beispiel 2

Herstellung eines Perlpolymerisats aus Methylmethacrylat, Divinylbenzol und Äthylvinylbenzol

| | |
|---|---|
| Lösung I (Dispergatorlösung | 2250 ml dest. Wasser<br>750 ml der 7,5 %igen Copolymerisatlösung gemäß Beispiel 1 |
| Lösung II | 685 g Methylmethacrylat<br>106,5 g Divinylbenzol (60 %ig in Äthylvinylbenzol)<br>3,75 g Benzoylperoxid<br>3,75 g Lauroylperoxid |

Polymerisation und Aufarbeitung wie in Beispiel 1
Ausbeute: 640 g,
mittlere Korngröße ca. 40 $\mu$.

## Beispiel 3

### Erfindungsgemäße, pastöse Dentalwerkstoffe

#### A) Peroxydpaste

320 g Perlpolymerisat aus Beispiel 1
112 g Bis-GMA
(Nupol 46 - 4005 der Firma Freeman Chemical)
68 g Triäthylenglycoldimethacrylat
3,6 g Benzoylperoxid

Die einzelnen Komponenten werden in einen Kneter gegeben und 60 Minuten lang intensiv geknetet, wobei während der letzten 10 Minuten ein Vakuum von ca. 20 Torr angelegt wird. Man erhält auf diese Weise eine knetbare Masse von besonders fester Konsistenz.

#### B) Aminpaste

Perlpolymerisat, Bis-GMA und Triäthylenglycoldimethacrylat werden in den gleichen Mengen wie bei der Peroxydpaste (A) eingesetzt und verarbeitet. Anstelle des Peroxids werden jedoch 3,6 g N,N-Bis-(2-hydroxypropyl)-3,5-dimethylanilin eingesetzt.

#### C) Pastöse Masse zum Füllen von Zähnen

Gleiche Teile (z.B. je 200 mg) der Amin- und Peroxydpaste werden 30 Sekunden lang intensiv gemischt.

Die erhaltene Mischung ist in hervorragender Weise als Zahnfüllmaterial geeignet. Sie härtet in wenigen Minuten unter geringem Polymerisationsschrumpf aus.

Beispiel 4

Erfindungsgemäßer, pastöser Dentalwerkstoff

Entsprechend der Arbeitsweise aus Beispiel 3 werden aus

330 g Perlpolymerisat gemäß Beispiel 2,
134 g Bis-GMA,
66 g Triäthylenglycoldimethacrylat und
4 g Benzoylperoxid bzw. 3 g N,N-Bis-(2-hydroxypropyl)-3,5-dimethylanalin

eine Amin- und eine Peroxydpaste hergestellt.

Dieses Material wird wie das Material aus Beispiel 3 gemischt. Man erhält ebenfalls eine Mischung, die in hervorragender Weise als Zahnfüllmaterial geeignet ist.

Beispiel 5

Erfindungsgemäßer, pastöser Dentalwerkstoff

Entsprechend der in Beispiel 3 angegebenen Arbeitsweise wird aus

300 g Perlpolymerisat gemäß Beispiel 1
112 g Bis GMA
68 g Triethylenglykoldimethacrylat und
0,9 g Benzoylperoxid

eine pastöse Mischung hergestellt.

Dieses Material ist zur Herstellung von künstlichen Zähnen geeignet, es läßt sich bei erhöhter Temperatur unter Formgebung aushärten. Geeignete Aushärtebedingungen sind 130°C/10 Min.

Beispiel 6

Erfindungsgemäßer, pastöser Pentalwerkstoff

Entsprechend der in Beispiel 3 angegebenen Arbeitsweise wird aus

260 g Perlpolymerisat gemäß Beispiel 1
80 g Triethylenglykoldimethacrylat
120 g Bis GMA und
4 g Benzoinisopropylether

eine pastöse Mischung hergestellt.

Dieses Material ist hervorragend als Zahnfüllungsmaterial geeignet. Es härtet unter Bestrahlung mit UV-Licht (Uviolite-Lampe der Firma Espe) innerhalb von 40 Sekunden in Schichtdicken von 3 mm aus.

Patentansprüche:

1. Dentalwerkstoffe auf Basis von organischen Kunststoffen in pastöser Form, dadurch gekennzeichnet, daß in ihnen mindestens eine Phase aus feinverteilten, vernetzten Kunststoffen besteht, die zu mindestens 50 % copolymerisierte Ester der Methacrylsäure enthalten, und die zweite Phase aus polymerisierbaren Bindern besteht, die zumindest 50 % Ester der Methacrylsäure enthalten.

2. Dentalwerkstoffe nach Anspruch 1, dadurch gekennzeichnet, daß die feinverteilten, vernetzten Kunststoffe vernetzte Polymerisatperlen mit einer mittleren Teilchengröße von 10 bis 100 /u sind.

3. Dentalwerkstoffe nach Anspruch 1, dadurch gekennzeichnet, daß sie 50-75 Gew.-% feinverteilte, vernetzte Kunststoffe enthalten.

4. Dentalwerkstoffe nach Anspruch 3, dadurch gekennzeichnet, daß die feinverteilten, vernetzten Kunststoffe zu mehr als 80 Gew.-% aus Estern der Methacrylsäure aufgebaut sind.

5. Dentalwerkstoffe nach Anspruch 1, dadurch gekennzeichnet, daß die zweite Phase zu mehr als 80 Gew.-% aus Estern der Methacrylsäure bestehen.